# EUROPÄISCHE PATENTSCHRIFT

(11) **EP 2 080 495 B1**
(45) Veröffentlichungstag und Bekanntmachung des Hinweises auf die Patenterteilung: **15.06.2011**
(21) Anmeldenummer: 08405021.0
(22) Anmeldetag: 21.01.2008
(51) Int. Cl.: A61F 9/008, A61F 9/009, G02B 7/16, G02B 21/24

(54) **Vorrichtung zur Bearbeitung von Augengewebe**
Device for processing eye tissue
Dispositif destiné au traitement de tissu oculaire

(43) Veröffentlichungstag der Anmeldung: 22.07.2009
(73) Patentinhaber: Ziemer Holding AG, 2562 Port (CH)
(72) Erfinder: Warturmer, Christian Rathjen, 28197 Bremen (DE)
(74) Vertreter: Vogel, Dany

(56) Entgegenhaltungen:
- EP-A- 1 486 185
- EP-A- 1 731 120
- DE-U1-202004 013 136
- US-A1- 2006 045 327
- US-A1- 2007 282 312

## Beschreibung

### Technisches Gebiet

Die vorliegende Erfindung betrifft eine Vorrichtung zur Bearbeitung von Augengewebe mittels Femtosekundenlaserpulsen. Die vorliegende Erfindung betrifft insbesondere eine Vorrichtung zur Bearbeitung von Augengewebe, welche ein optisches Übertragungssystem umfasst für die Übertragung von Femtosekundenlaserpulsen auf ein Projektionsobjektiv zum Projizieren der Femtosekundenlaserpulse auf respektive in das Augengewebe.

### Stand der Technik

Fehlsichtigkeiten wie Myopie (Kurzsichtigkeit), Hyperopie (Weitsichtigkeit oder Übersichtigkeit) oder Astigmatismus (Stabsichtigkeit) können heute durch refraktiv-chirurgische Behandlung dauerhaft korrigiert werden. Refraktiv-chirurgische Behandlungen sind chirurgische Eingriffe am Auge, die die optische Brechkraft des Auges ändern mit dem Ziel, diese einem gewünschten Wert möglichst gut anzunähern. Die Behandlung von Augengewebe z.B. für Gewebeschnitte und Gewebeabbau erfolgt heutzutage typischerweise mittels Lasertechnologie. Insbesondere werden für die Gewebebearbeitung stark fokussierte Femtosekundenlaserpulse verwendet, die Pulsbreiten von typisch 100fs bis 1000fs (1fs=10⁻¹⁵s) aufweisen. Zurzeit wird mit kommerziellen Femtosekundenlasersystemen die Comea (Homhaut) des Auges behandelt. Da mit den Laserpulsen aber auch andere Gewebeteile und Gewebebereiche des Auges behandelt werden, z.B. Sklera, Linse und Retina (Netzhaut), sind für die verschiedenen Anwendungsgebiete unterschiedliche Arbeitsabstände und damit Fokusabstände erforderlich, was neben mechanischen Positionierungsmechanismen auch unterschiedliche Lichtprojektionsoptiken erfordert. Zudem sind für unterschiedliche Anwendungen und Bearbeitungsgebiete oft auch unterschiedlich grosse und/oder verschieden gekrümmte Bildfelder (d.h. scharf fokussierte Bildbereiche) erforderlich, was wiederum mittels entsprechenden anwendungsspezifischen Projektionsoptiken erreicht werden kann. Um unterschiedliche anwendungspezifische Fokusdurchmesser, Fokusformen, Fokusausdehnungen in der Projektionsrichtung und/oder Strahldivergenzen zu erhalten, müssen ebenfalls verschiedene Projektionsoptiken mit jeweils unterschiedlicher Numerischer Apertur (NA) verwendet. Schliesslich können verschiedene Projektionsoptiken auch auf Grund der spezifischen optischen Eigenschaften von anzuwendenden Patientenschnittstellen erforderlich sein, insbesondere transparente Applikationselemente wie Kontaktkörper, z.B. Applanationskörper oder Distanzkörper. Eine flexible Anpassung der Projektionsoptik an die unterschiedlichen anwendungsspezifischen Anforderungen könnte idealerweise durch ein geeignetes Vario-Objektiv ermöglich werden.

Im Allgemeinen ist eine hohe NA wünschenswert, weil sich mit hoher NA kleine Brennpunkte (Spotgrösse) und damit eine kleinere Schnittzone pro Puls erzeugen lassen. Durch die oft gewünschten hohe NA, kurzen Arbeitsabstände und im Allgemeinen sehr kleinen Fokusgrössen (Spotgrössen) ist es tatsächlich äusserst schwierig Vario-Objektive herzustellen, die einen grossen Arbeitsbereich abdecken. Sind zusätzlich auch möglichst geringes Gewicht und kleine Baugrösse entscheidend, was beim Einsatz am Auge mit kurzem Arbeitsabstand durchaus der Fall ist, wird die Konstruktion von geeigneten Vario-Objektiven noch weiter erschwert. Unterschiedlich gekrümmte Bildfelder lassen sich mit im Stand der Technik bekannten Vario-Objektiven nicht realisieren. Da z.B. die Retina und die Cornea anders gekrümmt sind, sind auch andere Bildfeldwölbungen erforderlich.

US 2007/282312, EP 1731120 und EP 1486185 beschreiben jeweils Vorrichtungen zur Bearbeitung von Augengewebe mittels Femtosekundenlaserpulsen, welche ein optisches Übertragungssystem zur Übertragung der Femtosekundenlaserpulse auf ein Projektionsobjektiv zum Projizieren der Femtosekundenlaserpulse auf respektive in das Augengewebe umfassen.

US 2006/0045327 beschreibt eine Vorrichtung mit einer beweglichen Trägerplatte und darauf angebrachten unterschiedlichen Objektivlinse, wobei die Trägerplatte so verschiebbar ist, dass mit jeweils einer ausgewählten der Objektivlinsen ein Laserstrahl auf ein Objekt zugeführt werden kann.

DE 20 2004 013 136 U1 beschreibt eine Laseroptik mit einer Revolvereinheit zum rotierbaren Wechseln von verschiedenen Optikmodulen.

### Darstellung der Erfindung

Es ist eine Aufgabe der vorliegenden Erfindung eine Vorrichtung zur Bearbeitung von Augengewebe mittels Femtosekundenlaserpulsen, insbesondere mit Femtosekundenlaserpulsen, vorzuschlagen, welche nicht die Nachteile des Stands der Technik aufweist. Es ist insbesondere eine Aufgabe der vorliegenden Erfindung eine Vorrichtung zur Bearbeitung von Augengewebe mittels Femtosekundenlaserpulsen vorzuschlagen, welche eine flexible Anpassung der Projektionsoptik an unterschiedliche Anforderungen ermöglicht, ohne dazu aufwendige Vario-Objektive einsetzen zu müssen. Es ist insbesondere eine weitere Aufgabe der vorliegenden Erfindung eine Vorrichtung zur Bearbeitung von Augengewebe mittels Femtosekundenlaserpulsen vorzuschlagen, welche eine flexible Anpassung der Projektionsoptik an unterschiedliche Anforderungen von Brennweite (Fokusabstand), Bildfeldgrösse, Bildfeldkrümmung, Numerische Apertur, Fokusdurchmesser, Fokusform, Fokusausdehnung in Projektionsrichtung und/oder Strahldivergenz.

Gemäss der vorliegende Erfindung werden diese Ziele insbesondere durch die Elemente des unabhängigen Anspruchs 1 erreicht Weitere vorteilhafte Ausführungsformen gehen ausserdem aus den abhängigen Ansprüchen und der Beschreibung hervor.

Die Vorrichtung zur Bearbeitung von Augengewebe mittels Femtosekundenlaserpulsen umfasst ein optisches Übertragungssystem zur Übertragung der Femtosekundenlaserpulse auf ein Projektionsobjektiv, welches eingerichtet ist, die Femtosekundenlaserpulse auf respektive in das Augengewebe zu projizieren.

Die oben genannten Ziele werden durch die vorliegende Erfindung insbesondere dadurch erreicht, dass die Vorrichtung zur Bearbeitung von Augengewebe mittels Femtosekundenlaserpulsen zudem eine Objektivwechselvorrichtung umfasst, welche eingerichtet ist zum Auswechseln und Anschliessen des Projektionsobjektivs an das optische Übertragungssystem. Die Objektivwechselvorrichtung ermöglicht eine laserbasierte ophthalmologische Vorrichtung an neue Anwendungen anzupassen, ohne dazu grosse Umbauarbeiten vornehmen oder aufwendige Vario-Objektive einsetzen zu müssen. Die Objektivwechselvorrichtung erlaubt es dem Benutzer das Projektionsobjektiv flexibel und effizient zu wechseln, so dass das Projektionsobjektiv auch zwischen unterschiedlichen Anwendungen und Behandlungsschritten ohne grossen Zeitaufwand gewechselt werden kann. Insbesondere ermöglicht die Objektivwechselvorrichtung das System später an neue Anwendungen anzupassen, die zum Zeitpunkt der Herstellung der ophthalmologischen Vorrichtung noch nicht bekannt oder gebräuchlich waren. Gegenüber der Verwendung von Vario-Objektiven hat die Auswechslung von Projektionsobjektiven zudem den Vorteil, dass keine kontrollierte Einstellung von Objektivparametem erforderlich ist und somit damit verbundene Komponenten und Module für Parameterfeedback und Steuerung entfallen.

In einer bevorzugten Ausführungsvariante umfasst die Objektivwechselvorrichtung mehrere mechanisch miteinander verbundene unterschiedliche Projektionsobjektive, und die Objektivwechselvorrichtung ist eingerichtet, eines der Projektionsobjektive zum Anschliessen an das optische Übertragungssystem dem optischen Übertragungssystem zuzuführen. Die Projektionsobjektive unterscheiden sich zum Beispiel in ihrer Brennweite, Bildfeldgrösse, Bildfeldkrümmung, Numerischen Apertur, Fokusdurchmesser, Fokusform, Fokusausdehnung in Projektionsrichtung und/oder Strahldivergenz. In verschiedenen Ausführungsvarianten ist die Objektivwechselvorrichtung eingerichtet, die Projektionsobjektive durch Rotationsbewegungen oder Translationsbewegungen auszuwechseln, wobei jeweils eines der Projektionsobjektive zum Anschliessen an das optische Übertragungssystem durch eine Rotationsbewegung respektive eine Translationsbewegung dem optischen Übertragungssystem zugeführt wird. Eine Objektivwechselvorrichtung mit mehreren Projektionsobjektiven ermöglicht einen besonders effizienten Wechsel durch einfache Manipulation, ohne dass dazu während der Behandlung neue Projektionsobjektive an der ophthalmologischen Vorrichtung angebracht werden müssen.

In weiteren Ausführungsformen umfassen die Projektionsobjektive jeweils ein Applikationselement zur Applikation des Projektionsobjektivs an einem Auge, und/oder die Vorrichtung umfasst ein gemeinsames Applikationselement zur Applikation des Projektionsobjektivs an einem Auge, wobei die Objektivwechselvorrichtung eingerichtet ist, beim Anschliessen eines der Projektionsobjektive an das optische Übertragungssystem das betreffende Projektionsobjektiv mit dem Applikationselement zu kombinieren. Die Variante mit mehreren, jeweils an den Projektionsobjektiven angebrachten Applikationselementen hat den Vorteil, dass unabhängig vom Projektionsobjektiv auch unterschiedliche Applikationselemente, z.B. mit unterschiedlicher Kontaktform oder Arbeitsdistanz, durch einfache Manipulation ausgetauscht werden können. In einer Kombinationsausführung können sowohl unterschiedliche Applikationselemente und/oder Projektionsobjektive eingewechselt werden, als auch ein gemeinsames Applikationselement vorgesehen werden, das beispielsweise zur Fixierung am Auge dient.

In einer weiteren Ausführungsvariante umfasst die Objektivwechselvorrichtung ein Anschlussmodul zum entfernbaren Aufnehmen und Anschliessen des Projektionsobjektivs an das optische Übertragungssystem. Eine Kombination des Anschlussmoduls mit einer Objektivwechselvorrichtung für mehrere Projektionsobjektive ermöglicht die Objektivwechselvorrichtung vorbereitend auf eine Behandlung applikations- und/oder patientenspezifisch mit geeigneten Projektionsobjektiven und/oder Applikationselementen zu bestücken und dann während der Behandlung ohne weitere Manipulationen durch eine Rotations- oder Translationsbewegung einzuwechseln. Eine Objektivwechselvorrichtung mit bloss einem Anschlussmodul ermöglicht eine besonders einfach ausgestaltete Vorrichtung, die das Einsetzen und Auswechseln von verschiedenen Projektionsobjektiven während der Behandlung zulässt.

Vorzugsweise ist das optische Übertragungssystem eingerichtet, die Femtosekundenlaserpulse in im Wesentlichen parallelen Strahlen dem Projektionsobjektiv zuzuführen. Parallel in das Projektionsobjektiv eingehende Eingangsstrahlen haben den Vorteil, dass sich Ungenauigkeiten beim mechanischen Einwechseln von verschiedenen Projektionsobjektiven (Einbautoleranzen) nicht auf die durch das Projektionsobjektiv erzielte Fokustiefe auswirken.

In einer Ausführungsvariante umfasst die Vorrichtung ein Messsystem zur Bestimmung der Position des an das optische Übertragungssystem angeschlossenen Projektionsobjektivs relativ zur Vorrichtung. Das Messsystem ermöglicht die Detektion, Anzeige und/oder Korrektur von ungenau positionierten Projektionsobjektiven.

Gemäss der vorliegenden Erfindung umfasst die Vorrichtung einen Detektor zum Bestimmen einer Objektivtypkennung, mit welcher das an das optische Übertragungssystem angeschlossene Projektionsobjektiv versehen ist. Vorzugsweise ist der Detektor mit einem Lasersteuermodul verbunden und ist eingerichtet, dem Lasersteuermodul die Objektivtypkennung des an das optische Übertragungssystem angeschlossenen Projektionsobjektivs zu übermitteln. Die Objektivtypkennung ist beispielsweise als mechanische, optische, elektrische oder funkbasierte Kennung ausgeführt. Durch die Zuordnung und Erkennung eines Objektivtyps kann der Laserstrahl in seiner Beschaffenheit, Übertragung, Ausrichtung und/oder Ablenkung abhängig von den optischen Eigenschaften des verwendeten Projektionsobjektivs gesteuert werden.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1a zeigt ein Blockdiagramm, welches schematisch eine ophthalmologische Vorrichtung zur Behandlung von Augengewebe mittels Femtosekundenlaserpulsen darstellt.
Figur 1b zeigt eine Aufsicht einer durch die ophthalmologische Vorrichtung bearbeiteten Bearbeitungsfläche im Augengewebe.
Figur 2 zeigt ein Blockdiagramm, welches schematisch eine Ausführungsvariante der ophthalmologischen Vorrichtung mit mehreren mechanisch verbundenen Projektionsobjektiven in der Ansicht darstellt.
Figur 3 zeigt ein Blockdiagramm, welches schematisch eine weitere Ausführungsvariante der ophthalmologischen Vorrichtung mit mehreren mechanisch verbundenen Projektionsobjektiven in der Aufsicht darstellt.
Figur 4a zeigt ein Blockdiagramm, welches schematisch eine weitere Ausführungsvariante der ophthalmologischen Vorrichtung mit mehreren jeweils an einem der Projektionsobjektive angebrachten Applikationselemente darstellt.
Figur 4b zeigt ein Blockdiagramm, welches schematisch eine weitere Ausführungsvariante der ophthalmologischen Vorrichtung mit einem für mehrere Projektionsobjektive gemeinsamen Applikationselement darstellt.
Figur 4c zeigt ein Blockdiagramm, welches schematisch eine kombinierte Ausführungsvariante der ophthalmologischen Vorrichtung mit mehreren an den Projektionsobjektiven angebrachten Applikationselementen und einem gemeinsamen weiteren Applikationselement darstellt.

### Wege zur Ausführung der Erfindung

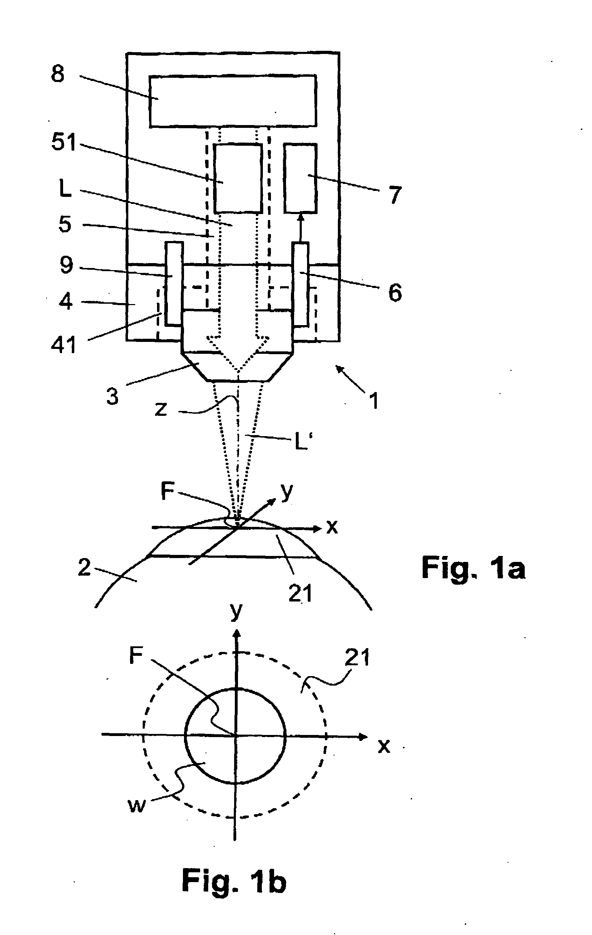

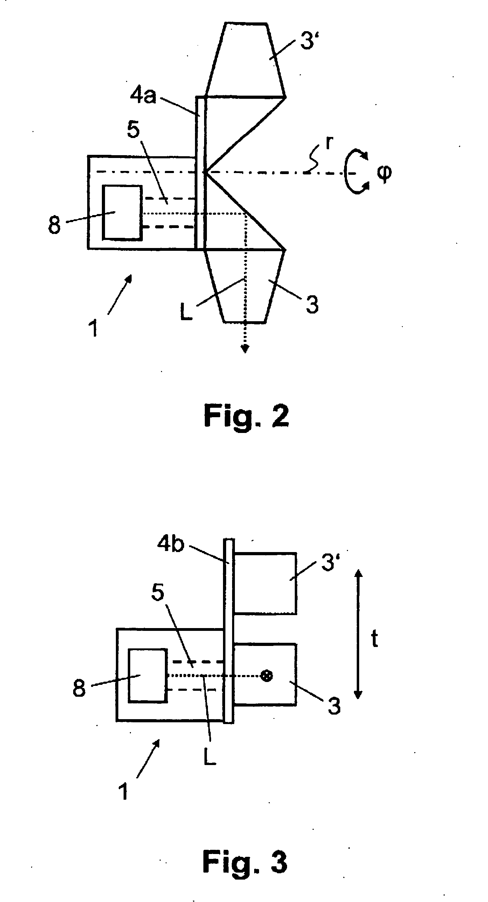

In den Figuren 1a, 2, 3, 4a, 4b und 4c bezeichnet das Bezugszeichen 1 eine ophthalmologische Vorrichtung, respektive eine ophthalmologische Vorrichtungsanordnung, mit einer Laserquelle 8 und einem optischen Übertragungssystem 5, welches die Laserquelle 8 zur fokussierten Projektion von Laserpulsen optisch mit einem (Licht-) Projektionsobjektiv 3 verbindet. Die Laserquelle 8 umfasst insbesondere einen Femtosekundenlaser zur Erzeugung eines Laserstrahls L mit Femtosekundenlaserpulsen, die Pulsbreiten von typisch 10fs bis 1000fs (1fs=10⁻¹⁵s) aufweisen. Die Laserquelle 8 ist in einem separaten oder in einem mit dem Projektionsobjektiv 3 gemeinsamen Gehäuse angeordnet. Das Projektionsobjektiv 3 projiziert einen fokussierten gepulsten Laserstrahl L' für die punktuelle Gewebeauflösung in einem Fokus F (Brennpunkt) auf oder im Innern des Augengewebes 21, beispielsweise auf oder in der Hornhaut (Cornea). Wie später detaillierter beschrieben wird, umfasst die ophthalmologische Vorrichtung 1 in den Ausführungsvarianten nach Figuren 2, 3, 4a, 4b und 4c mehrere (aus-)wechselbare Projektionsobjektive 3, 3'.

Das Übertragungssystem 5 umfasst mehrere optische Elemente, wie Linsen, Blenden, Umlenkspiegel und Lichtleiter, zur Zuführung der Femtosekundenlaserpulse von der Laserquelle 8 zum angeschlossenen Projektionsobjektiv 3. Das Übertragungssystem 5 ist vorzugsweise eingerichtet die Laserstrahlen L im Wesentlichen parallel dem Projektionsobjektiv 3 zuzuführen, was beispielsweise bei Wechselobjektiven in der Photographie nicht der Fall ist. Wie in der Figur 1a zudem schematisch dargestellt ist, umfasst das optische Übertragungssystem 5 ein Ablenkungsmodul 51, d.h. ein optisches Abtastmodul oder Scannermodul, welches eingerichtet ist, die von der Laserquelle 8 erzeugten Femtosekundenlaserpulse in mindestens einer Richtung abzulenken und dadurch den Fokus F des gepulsten Laserstrahls L' entsprechend einem Abtastmuster (Scanpattem) mindestens in einer Richtung x, y der (zusammenhängenden oder nicht zusammenhängenden) definierten Bearbeitungsfläche w im Gewebe 21 des Auges 3 zu bewegen. Das Ablenkungsmodul 51 ist, beispielsweise zusammen mit der Laserquelle 8, in einem separaten oder in einem mit dem Projektionsobjektiv 3 respektive Objektivwechselvorrichtung 4 gemeinsamen Gehäuse angeordnet. In einer Ausführungsvariante umfasst das optische Übertragungssystem 5 und/oder das Projektionsobjektiv 3 bewegliche Linsen, um den Fokus F des gepulsten Laserstrahls L' auch in einer zu den x/y-Richtungen Normalen (z.B. entlang der optischen Achse z) zu verstellen. Je nach Ausführungsvariante umfasst die ophthalmologische Vorrichtung 1 zudem optional ein Antriebsmodul um das Projektionsobjektiv 3 und damit den Fokus F entlang den Richtungen x und/oder y und/oder der Normalen zu bewegen.

Zum besseren Verständnis soll hier angeführt werden, dass die Figuren 1a, 2, 3, 4a, 4b und 4c die ophthalmologische Vorrichtung 1 schematisch und vereinfacht darstellen. Zum Beispiel ist in den Figuren nicht präzise wiedergegeben, dass die Projektionsobjektive 3, 3' eine hohe numerische Apertur von beispielsweise mindestens 0.3 aufweisen.

In der Figur 1a bezeichnet das Bezugszeichen 4 eine Objektivwechselvorrichtung zum Auswechseln und Anschliessen des Projektionsobjektivs 3 an das optische Übertragungssystem 5. In den Figuren 1a, 2, 3 4a, 4b und 4c sind verschiedene Ausführungsformen der Objektivwechselvorrichtung 4 dargestellt.

In der Ausführung nach Figur 1a umfasst die Objektivwechselvorrichtung 4 ein Anschlussmodul 41 zum entfernbaren Einsetzen, Aufnehmen und Anschliessen des Projektionsobjektivs 3 an das optische Übertragungssystem 5. Das Anschlussmodul 41 umfasst beispielsweise ein Gewinde- oder einen Bajonettverschluss zum Aufnehmen und Befestigen des Projektionsobjektivs 3 an der ophthalmologischen Vorrichtung 1. Im befestigten Zustand ist das Projektionsobjektiv 3 mit dem optischen Übertragungssystem 5 optisch verbunden.

In den Ausführungen gemäss den Figuren 2, 3 4a, 4b und 4c umfasst die Objektivwechselvorrichtung 4, 4a, 4b jeweils mehrere mechanisch miteinander verbundene unterschiedliche Projektionsobjektive 3, 3'. Die Projektionsobjektive 3, 3' sind jeweils mit einer Objektivtypkennung versehen und unterscheiden sich durch ihre optischen Eigenschaften wie Numerische Apertur, Brennweite, Bildfeldgrösse, Bildfeldkrümmung, Fokusdurchmesser, Fokusform, Fokusausdehnung in Projektionsrichtung und/oder Strahldivergenz.

In der Ausführung nach Figur 2 ist die Objektivwechselvorrichtung 4a eingerichtet, die Projektionsobjektive 3, 3' durch Rotationsbewegungen ϕ um eine Drehachse r auszuwechseln. Die Objektivwechselvorrichtung 4a umfasst einen um die Drehachse r rotierbaren Träger, an welchem die Projektionsobjektive 3, 3' befestigt sind. Die Objektivwechselvorrichtung 4a ist beispielsweise in Form einer Revolveroptik ausgeführt. Durch eine Rotationsbewegung ϕ wird ein ausgewähltes Projektionsobjektiv 3, 3' dem optischen Übertragungssystem 5 zugeführt und mit dem optischen Übertragungssystem 5 optisch verbunden.

In der Ausführung nach Figur 3 ist die Objektivwechselvorrichtung 4b eingerichtet, die Projektionsobjektive 3, 3' durch Translationsbewegungen t auszuwechseln. Die Objektivwechselvorrichtung 4b umfasst einen entlang einer Achse verschiebbaren Trägerschlitten, an welchem die Projektionsobjektive 3, 3' befestigt sind. Durch eine Translationsbewegung t wird ein ausgewähltes der Projektionsobjektive 3, 3' dem optischen Übertragungssystem 5 zugeführt und mit dem optischen Übertragungssystem 5 optisch verbunden.

Das ausgewählte Projektionsobjektiv 3 wird vorzugsweise beim Anschliessen an das optische Übertragungssystem 5 in der Rotation respektive Translation fixiert, beispielsweise mechanisch durch einen Einrast- oder Anschlagmechanismus.

In einer Variante weist der Träger der Objektivwechselvorrichtung 4a, 4b ein oder mehrere Anschlussmodule zum entfernbaren Aufnehmen jeweils eines Projektionsobjektivs 3, 3' auf, wie im Zusammenhang mit Figur 1a beschrieben wurde. Dadurch kann der Objektivträger mit unterschiedlichen Sätzen von Projektionsobjektiven 3, 3' bestückt werden, die beispielsweise für verschiedene Behandlungsschritte und Anwendungen während einer Behandlung eines Patienten vorgesehen sind.

Wie in der Figur 1 a schematisch dargestellt ist, umfasst die ophthalmologische Vorrichtung 1 einen Detektor 6 zum Detektieren der Objektivtypkennung des angeschlossenen Projektionsobjektivs 3. Die Objektivtypkennung ist beispielsweise als mechanische, optische, elektrische oder funkbasierte Kennung ausgeführt, welche eine dem Objektivtyp zugeordnete Typencodierung angibt. Entsprechend der Ausführung der Objektivtypkennung umfasst der Detektor 6 einen Sensor zum Detektieren und Lesen einer mechanisch ausgebildeten Kennung, z.B. ein durch Strukturelemente gebildeter Code, zum Erfassen einer optisch ausgebildeten Kennung, z.B. ein Strichcode oder eine Lichtblendencodierung, zum Lesen einer elektrisch ausgebildeter Kennung, z.B. ein kapazitiver oder ohmscher Code, oder zum Empfanden und Erkennen einer funkbasierten Kennung, z.B. eine RFID (Radio Frequency Identification).

Der Detektor 6 ist mit einem Lasersteuermodul 7 verbunden, das als programmiertes Logikmodul mittels Software und/oder Hardware ausgeführt ist, und ist eingerichtet die bestimmte Objektivtypkennung an das Lasersteuermodul 7 zu übermitteln. Das Lasersteuermodul 7 ist zur Übertragung von Steuersignalen (Steuerbefehle, Steuerprogramme) mit dem Ablenkungsmodul 51 und der Laserquelle 8 verbunden. Das Lasersteuermodul 7 ist in einem separaten oder in einem mit der Objektivwechselvorrichtung 4 gemeinsamen Gehäuse angeordnet. Das Lasersteuermodul 7 umfasst für verschiedene Objektivtypen jeweils zugeordnete physikalische Nennwerte, welche optische Eigenschaften des betreffenden Objektivtyps, z.B. Numerische Apertur, Brennweite, Bildfeldgrösse, Bildfeldkrümmung, Fokusdurchmesser, Fokusform, Fokusausdehnung in Projektionsrichtung und/oder Strahldivergenz, und/oder zugeordnete Steuerprogrammmodule angeben. Das Lasersteuermodul 7 ist eingerichtet, die Laserquelle 8 und das optische Übertragungssystem 5, insbesondere das Ablenkungsmodul 51, basierend auf der detektierten Objektivtypkennung zu steuern, indem Steuerbefehle aus zugeordneten Steuerprogrammmodulen und/oder in Abhängigkeit der zugeordneten physikalischen Nennwerte an die Laserquelle 8, das Ablenkungsmodul 51 und/oder andere steuerbare Elemente des optischen Übertragungssystems 5 übermittelt werden, z.B. verschiebbare Linsen und steuerbare Blenden. Somit wird der Laserstrahl L der Laserquelle 8, z.B. seine Energiehöhe, Pulsrate, und/oder Pulsbreite, sowie dessen Übertragung, Ausrichtung und/oder Ablenkung abhängig vom verwendeten Objektivtyp automatisch verändert und angepasst.

In der Figur 1a bezeichnet das Bezugszeichen 9 ein Messsystem zur Bestimmung der Position des an das optische Übertragungssystem 5 angeschlossenen Projektionsobjektivs relativ zur ophthalmologischen Vorrichtung 1 und insbesondere relativ zum optischen Übertragungssystem 5. In verschiedenen Ausführungsvarianten ist das Messsystem 9 eingerichtet die mechanische Relativposition kapazitiv, induktiv, ohmsch oder optisch zu ermitteln. Das Messsystem 9 ist mit dem Lasersteuermodul 7 verbunden und ist eingerichtet, die ermittelte Relativposition an das Lasersteuermodul 7 zu übermitteln. Abhängig von der Ausführungsvariante zeigt das Lasersteuermodul 7 dem Benutzer eine Abweichung der Relativposition von einem definierten Toleranzbereich über eine Benutzerschnittstelle an, z.B. akustisch und/oder optisch, und/oder passt die Steuerung der Laserquelle 8 und/oder des optischen Übertragungssystems - 5, insbesondere des Ablenkungsmoduls 51, entsprechend der Abweichung von einer definierten Sollposition an.

Obwohl dies in den vereinfachten Figuren 2, 3, 4a, 4b und 4c nicht explizit dargestellt ist, umfasst die in diesen Figuren dargestellte ophthalmologische Vorrichtung 1 in entsprechenden Ausführungsvarianten ebenso ein Lasersteuermodul 7 sowie einen Detektor 6 und/oder ein Messsystem 9 zur Typen- respektive Positionsbestimmung und darauf basierten Steuerung des Laserstrahls L.

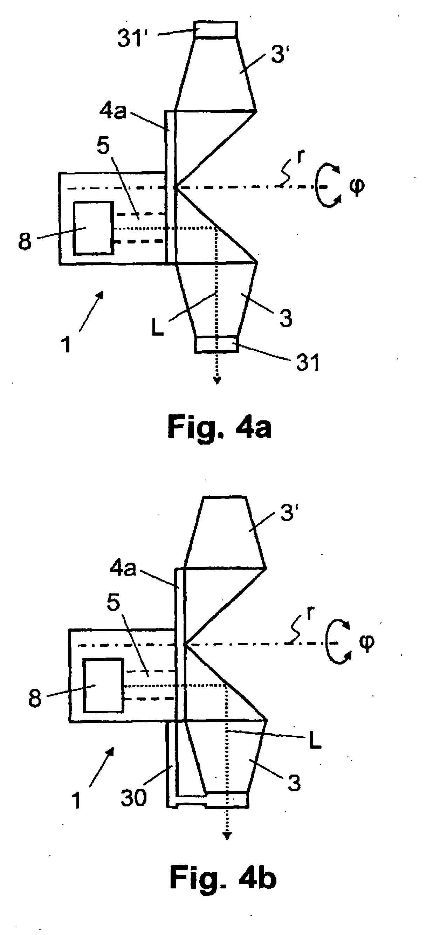

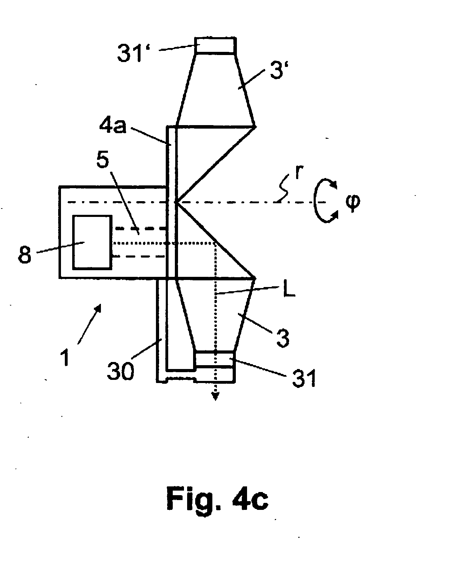

Die Figuren 4a, 4b und 4c zeigen Ausführungsvarianten, in welchen die ophthalmologische Vorrichtung 1 mit einem oder mehreren Applikationselementen 30, 31, 31' versehen ist. Die Applikationselemente 30, 31, 31' umfassen beispielsweise Kontaktkörper, z.B. mindestens stellen- und/oder teilweise transparente Applanationskörper oder konkave/konvexe Formkörper. Je nach Ausführungsvariante umfassen die Applikationselemente 30, 31, 31' zudem einen Saugring, oder andere Befestigungsvorrichtungen, zur Fixierung am Auge 2.

In der Ausführung nach Figur 4a sind die Applikationselemente 31, 31' jeweils fest oder austauschbar an den Projektionsobjektiven 3, 3' angebracht und können beispielsweise unterschiedlich ausgestaltet sein, z.B. ein Applanationskörper, ein konkaver Formkörper, unterschiedliche Distanzkörper für verschiedene Behandlungsschritte und/oder mit, ohne oder unterschiedliche Befestigungsmittel zur Fixierung am Auge 2.

In der Ausführung nach Figur 4b, ist die ophthalmologische Vorrichtung 1 mit einem gemeinsamen festen oder austauschbaren Applikationselement 30 versehen, und die Objektivwechselvorrichtung 4 ist so eingerichtet und angeordnet, dass die verschiedenen Projektionsobjektive 3, 3' so gewechselt werden können, dass sie im am optischen Übertragungssystem 5 angeschlossenen Zustand berührungsfrei oder mechanisch kontaktierend mit dem Applikationselement 30 kombiniert werden.

Die Ausführung nach Figur 4c ist eine Kombination der Ausführungen nach den Figuren 4a und 4b, wobei einerseits jeweils verschiedene Applikationselemente 31, 31' an den Projektionsobjektiven 3, 3' angebracht sind und andererseits ein gemeinsames Applikationselement 30 an der ophthalmologischen Vorrichtung 1 vorgesehen ist. In der kombinierten Ausführung nach Figur 4c können während einer Behandlung einerseits unterschiedliche Projektionsobjektive 3, 3' und/oder Applikationselemente 31, 31' selektiert und eingewechselt werden und andererseits ein gemeinsames Applikationselement 30 während der Behandlung beibehalten werden, beispielsweise ein Saugring zur Befestigung am Auge 2, ein Distanzkörper und/oder eine Schutzblende.

Obwohl dies nicht dargestellt ist, sind die Applikationselemente 30, 31, 31' auch mit den Ausführungen gemäss den Figuren 1a und 3 kombinierbar.

In einer weiteren Ausführungsvariante umfasst die Objektivwechselvorrichtung 4, 4a, 4b zudem ein optionales Antriebsmodul zum motorisierten Wechseln der Projektionsobjektiven 3, 3' und/oder. Applikationselemente 31, 31'.

## Patentansprüche

1. Vorrichtung (1) zur Bearbeitung von Augengewebe (21) mittels Femtosekundenlaserpulsen umfassend ein optisches Übertragungssystem (5) zur Übertragung der Femtosekundenlaserpulse auf ein Projektionsobjektiv (3) zum Projizieren der Femtosekundenlaserpulse auf respektive in das Augengewebe (21), **gekennzeichnet durch**
eine Objektivwechselvorrichtung (4), welche eingerichtet ist zum Auswechseln und Anschliessen des Projektionsobjektivs (3) an das optische Übertragungssystem (5), und
einen Detektor (6) zum Bestimmen einer Objektivtypkennung, mit welcher das an das optische Übertragungssystem (5) angeschlossene Projektionsobjektiv (3) versehen ist, wobei der Detektor (6) mit einem Lasersteuermodul (7) verbunden ist, und wobei der Detektor (6) eingerichtet ist, dem Lasersteuermodul (7) die Objektivtypkennung des an das optische Übertragungssystem (5) angeschlossenen Projektionsobjektivs (3) zu übermitteln.

2. Vorrichtung (1) nach Anspruch 1, **dadurch gekennzeichnet, dass** die Objektivwechselvorrichtung (4) mehrere mechanisch miteinander verbundene unterschiedliche Projektionsobjektive (3, 3') umfasst, und dass die Objektivwechselvorrichtung (4) eingerichtet ist, eines der Projektionsobjektive (3, 3') zum Anschliessen an das optische Übertragungssystem (5) dem optischen Übertragungssystem (5) zuzuführen.

3. Vorrichtung (1) nach Anspruch 2, **dadurch gekennzeichnet, dass** die Objektivwechselvorrichtung (4a) eingerichtet ist, die Projektionsobjektive (3, 3') durch Rotationsbewegungen (ϕ) auszuwechseln, wobei jeweils eines der Projektionsobjektive (3, 3') zum Anschliessen an das optische Übertragungssystem (5) durch eine Rotationsbewegung (ϕ) dem optischen Übertragungssystem (5) zugeführt wird.

4. Vorrichtung (1) nach Anspruch 2, **dadurch gekennzeichnet, dass** die Objektivwechselvorrichtung (4b) eingerichtet ist, die Projektionsobjektive (3, 3') durch Translationsbewegungen (t) auszuwechseln, wobei jeweils eines der Projektionsobjektive (3, 3') zum Anschliessen an das optische Übertragungssystem (5) durch eine Translationsbewegung (t) dem optischen Übertragungssystem (5) zugeführt wird.

5. Vorrichtung (1) nach einem der Ansprüche 2 bis 4, **dadurch gekennzeichnet, dass** die Projektionsobjektive (3, 3') sich in mindestens einer Eigenschaft aus Brennweite, Bildfeldgrösse, Bildfeldkrümmung, Numerische Apertur, Fokusdurchmesser, Fokusform, Fokusausdehnung in Projektionsrichtung und Strahldivergenz unterscheiden.

6. Vorrichtung (1) nach einem der Ansprüche 2 bis 5, **dadurch gekennzeichnet, dass** die Projektionsobjektive (3, 3') jeweils ein Applikationselement (31, 31') umfassen zur Applikation des Projektionsobjektivs (3, 3') an einem Auge (2).

7. Vorrichtung (1) nach einem der Ansprüche 2 bis 6, **dadurch gekennzeichnet, dass** die Vorrichtung (1) ein Applikationselement (30) umfasst zur Applikation des Projektionsobjektivs (3) an einem Auge (2), und dass die Objektivwechselvorrichtung (4) eingerichtet ist, beim Anschliessen eines der Projektionsobjektive (3) an das optische Übertragungssystem (5) das betreffende Projektionsobjektiv (3) mit dem Applikationselement (30) zu kombinieren.

8. Vorrichtung (1) nach einem der Ansprüche 1 bis 7, **dadurch gekennzeichnet, dass** die Objektivwechselvorrichtung (4) mindestens ein Anschlussmodul (41) zum entfernbaren Aufnehmen und Anschliessen des Projektionsobjektivs (3) an das optische Übertragungssystem (5) umfasst.

9. Vorrichtung (1) nach einem der Ansprüche 1 bis 8, **dadurch gekennzeichnet, dass** das optische Übertragungssystem (5) eingerichtet ist, die Femtosekundenlaserpulse in im Wesentlichen parallelen Strahlen dem Projektionsobjektiv (3) zuzuführen.

10. Vorrichtung (1) nach einem der Ansprüche 1 bis 9, **dadurch gekennzeichnet, dass** das Lasersteuermodul (7) eingerichtet ist, das optische Übertragungssystem (5) basierend auf der Objektivtypkennung zu steuern und somit einen Laserstrahl (L) mindestens hinsichtlich eines aus seiner Übertragung, seiner Ausrichtung und seiner Ablenkung abhängig vom verwendeten Objektivtyp automatisch anzupassen.

11. Vorrichtung (1) nach einem der Ansprüche 1 bis 10, **dadurch gekennzeichnet, dass** das Lasersteuermodul (7) eingerichtet ist, basierend auf der Objektivtypkennung eine Laserquelle (8) zu steuern und somit einen Laserstrahl (L) der Laserquelle (8) mindestens hinsichtlich eines aus seiner Energiehöhe, seiner Pulsrate und seiner Pulsbreite abhängig vom verwendeten Objektivtyp automatisch anzupassen.

12. Vorrichtung (1) nach einem der Ansprüche 1 bis 11, **dadurch gekennzeichnet, dass** das Lasersteuermodul (7) eingerichtet ist, basierend auf der Objektivtypkennung Steuerbefehle an mindestens eines aus einer Laserquelle (8), einem vom Übertragungssystem (5) umfasstes Ablenkungsmodul (51), anderer steuerbarer Elemente des optischen Übertragungssystems (5), insbesondere eine verschiebbare Linse und eine steuerbare Blende zu übermitteln.

13. Vorrichtung (1) nach Anspruch 12, **dadurch gekennzeichnet, dass** das Lasersteuermodul (7) für verschiedene Objektivtypen jeweils zugeordnete physikalische Nennwerte umfasst, welche optische Eigenschaften des betreffenden Objektivtyps angeben, und dass das Lasersteuermodul (7) eingerichtet ist, Steuerbefehle in Abhängigkeit der zugeordneten physikalischen Nennwerte zu übermitteln.

14. Vorrichtung (1) nach einem der Ansprüche 1 bis 13, **dadurch gekennzeichnet, dass** die Objektivtypkennung ausgeführt ist als eine aus mechanische, optische, elektrische und funkbasierte Kennung.

15. Vorrichtung (1) nach einem der Ansprüche 1 bis 14, **gekennzeichnet durch** ein Messsystem (9) zur Bestimmung der Position des an das optische Übertragungssystem (5) angeschlossenen Projektionsobjektivs (3) relativ zur Vorrichtung (1).

## Claims

1. Apparatus (1) for processing of eye tissue (21) by means of femtosecond laser pulses comprising an optical transmission system (5) for transmission of femtosecond laser pulses to a projection objective (3) for projection of the femtosecond laser pulses onto or into the eye tissue (21),
**characterized by**
an objective changing apparatus (4), which is designed to replace the projection objective (3) and to connect it to the optical transmission system (5), and
a detector (6) for determination of an objective type identification, with which the projection objective (3) which is connected to the optical transmission system (5) is provided, with the detector (6) being connected to a laser control module (7), and with the detector (6) being designed to transmit to the laser control module (7) the objective type identification of the projection objective (3) which is connected to the optical transmission system (5).

2. Apparatus (1) according to Claim 1, **characterized in that** the objective changing apparatus (4) comprises a plurality of different projection objectives (3, 3') which are mechanically connected to one another, and **in that** the objective changing apparatus (4) is designed to feed to the optical transmission system (5) one of the projection objectives (3, 3') for connection to the optical transmission system (5).

3. Apparatus (1) according to Claim 2, **characterized in that** the objective changing apparatus (4a) is designed to replace the projection objectives (3, 3') by rotary movements (ϕ), with in each case one of the projection objectives (3, 3') being fed to the optical transmission system (5) by a rotary movement (ϕ) for connection to the optical transmission system (5).

4. Apparatus (1) according to Claim 2, **characterized in that** the objective changing apparatus (4b) is designed to replace the projection objectives (3, 3') by translation movements (t), with in each case one of the projection objectives (3, 3') being fed to the optical transmission system (5) by a translation movement (t) for connection to the optical transmission system (5).

5. Apparatus (1) according to one of Claims 2 to 4, **characterized in that** the projection objectives (3, 3') differ in at least one characteristic of focal length, image field size, image field curvature, numerical aperture, focus diameter, focus form, focus extent in the projection direction and beam divergence.

6. Apparatus (1) according to one of Claims 2 to 5, **characterized in that** the projection objectives (3, 3') each comprise an application element (31, 31') for application of the projection objective (3, 3') to an eye (2).

7. Apparatus (1) according to one of Claims 2 to 6, **characterized in that** the apparatus (1) comprises an application element (30) for application of the projection objective (3) to an eye (2), and **in that** the objective changing apparatus (4) is designed to combine the relevant projection objective (3) with the application element (30) when one of the projection objectives (3) is connected to the optical transmission system (5).

8. Apparatus (1) according to one of Claims 1 to 7, **characterized in that** the objective changing apparatus (4) comprises at least one connecting module (41) for removable holding and connection of the projection objective (3) to the optical transmission system (5).

9. Apparatus (1) according to one of Claims 1 to 8, **characterized in that** the optical transmission system (5) is designed to feed the femtosecond laser pulses in substantially parallel beams to the projection objective (3).

10. Apparatus (1) according to one of Claims 1 to 9, **characterized in that** the laser control module (7) is designed to control the optical transmission system (5) on the basis of the objective type identification, and thus to automatically adapt a laser beam (L), at least with respect to one of its transmission, its alignment and its deflection, depending on the objective type used.

11. Apparatus (1) according to one of Claims 1 to 10, **characterized in that** the laser control module (7) is designed to control a laser source (8) on the basis of the objective type identification, and thus to automatically adapt a laser beam (L) from the laser source (8) at least with respect to one of its energy level, its pulse rate and its pulse width, depending on the objective type used.

12. Apparatus (1) according to one of Claims 1 to 11, **characterized in that** the laser control module (7) is designed to transmit, on the basis of the objective type identification, control commands to at least one of a laser source (8), a deflection module (51) which is comprised by the transmission system (5), other controllable elements of the optical transmission system (5), in particular a movable lens and a controllable aperture.

13. Apparatus (1) according to Claim 12, **characterized in that** the laser control module (7) comprises respectively associated physical nominal values for different objective types, which nominal values indicate optical characteristics of the relevant objective type, and **in that** the laser control module (7) is designed to transmit control commands as a function of the associated physical nominal values.

14. Apparatus (1) according to one of Claims 1 to 13, **characterized in that** the objective type identification is in the form of one of mechanical, optical, electrical and radio-based identification.

15. Apparatus (1) according to one of Claims 1 to 14, **characterized by** a measurement system (9) for determination, relative to the apparatus (1), of the position of the projection objective (3) which is connected to the optical transmission system (5).

## Revendications

1. Dispositif (1) de traitement de tissu oculaire (21) au moyen d'impulsions laser femtosecondes, comprenant un système de transmission optique (5) pour transmettre l'impulsion laser femtoseconde sur un objectif de projection (3) afin de projeter l'impulsion laser femtoseconde sur ou dans le tissu oculaire (21),
**caractérisé par**
un dispositif de changement d'objectif (4) qui est conçu pour remplacer et raccorder l'objectif de projection (3) au système de transmission optique (5) et
un détecteur (6) pour déterminer un identifiant de type d'objectif dont est muni l'objectif de projection (3) raccordé au système de transmission optique (5), le détecteur (6) étant relié avec un module de commande de laser (7) et le détecteur (6) étant conçu pour communiquer au module de commande de laser (7) l'identifiant de type d'objectif de l'objectif de projection (3) raccordé au système de transmission optique (5).

2. Dispositif (1) selon la revendication 1, **caractérisé en ce que** le dispositif de changement d'objectif (4) comprend plusieurs objectifs de projection (3, 3') différents reliés mécaniquement entre eux et **en ce que** le dispositif de changement d'objectif (4) est conçu pour acheminer au système de transmission optique (5) l'un des objectifs de projection (3, 3') en vue de son raccordement au système de transmission optique (5).

3. Dispositif (1) selon la revendication 2, **caractérisé en ce que** le dispositif de changement d'objectif (4a) est conçu pour remplacer les objectifs de projection (3, 3') par des mouvements de rotation (ϕ), l'un des objectifs de projection (3, 3') étant à chaque fois acheminé au système de transmission optique (5) par un mouvement de rotation (ϕ) en vue de son raccordement au système de transmission optique (5).

4. Dispositif (1) selon la revendication 2, **caractérisé en ce que** le dispositif de changement d'objectif (4b) est conçu pour remplacer les objectifs de projection (3, 3') par des mouvements de translation (t), l'un des objectifs de projection (3, 3') étant à chaque fois acheminé au système de transmission optique (5) par un mouvement de translation (t) en vue de son raccordement au système de transmission optique (5).

5. Dispositif (1) selon l'une des revendications 2 à 4, **caractérisé en ce que** les objectifs de projection (3, 3') se différentient au moins au niveau d'une propriété parmi la distance focale, la taille du champ d'image, la courbure du champ d'image, l'ouverture numérique, le diamètre du foyer, la forme du foyer, l'expansion du foyer dans le sens de la projection et la divergence des rayons.

6. Dispositif (1) selon l'une des revendications 2 à 5, **caractérisé en ce que** les objectifs de projection (3, 3') comprennent respectivement un élément d'application (31, 31') pour appliquer l'objectif de projection (3, 3') sur un oeil (2).

7. Dispositif (1) selon l'une des revendications 2 à 6, **caractérisé en ce que** le dispositif (1) comprend un élément d'application (30) pour appliquer l'objectif de projection (3) sur un oeil (2) et **en ce que** le dispositif de changement d'objectif (4) est conçu, lors du raccordement de l'un des objectifs de projection (3) au système de transmission optique (5), pour combiner l'objectif de projection (3) concerné avec l'élément d'application (30).

8. Dispositif (1) selon l'une des revendications 1 à 7, **caractérisé en ce que** le dispositif de changement d'objectif (4) comprend au moins un module de raccordement (41) pour accueillir de manière amovible et raccorder l'objectif de projection (3) au système de transmission optique (5).

9. Dispositif (1) selon l'une des revendications 1 à 8, **caractérisé en ce que** le système de transmission optique (5) est conçu pour acheminer l'impulsion laser femtoseconde à l'objectif de projection (3) dans des rayons pour l'essentiel parallèles.

10. Dispositif (1) selon l'une des revendications 1 à 9, **caractérisé en ce que** le module de commande de laser (7) est conçu pour commander le système de transmission optique (5) en se basant sur l'identifiant de type d'objectif et pour adapter ainsi automatiquement un rayon laser (L) en fonction du type d'objectif utilisé au moins du point de vue de l'une parmi sa transmission, son orientation et sa déviation.

11. Dispositif (1) selon l'une des revendications 1 à 10, **caractérisé en ce que** le module de commande de laser (7) est conçu pour commander une source laser (8) en se basant sur l'identifiant de type d'objectif et pour adapter ainsi automatiquement un rayon laser (L) de la source laser (8) en fonction du type d'objectif utilisé au moins du point de vue de l'un/une parmi son niveau d'énergie, sa fréquence d'impulsions et sa largeur d'impulsions.

12. Dispositif (1) selon l'une des revendications 1 à 11, **caractérisé en ce que** le module de commande de laser (7) est conçu pour, en se basant sur l'identifiant de type d'objectif, communiquer des instructions de commande à au moins un/une parmi une source laser (8), un module de déviation (51) inclus dans le système de transmission (5), d'autres éléments commandables du système de transmission optique (5), notamment une lentille coulissante et un obturateur commandable.

13. Dispositif (1) selon la revendication 12, **caractérisé en ce que** le module de commande de laser (7) inclut des valeurs nominales physiques respectivement associées à différents types d'objectifs, lesquelles indiquent des caractéristiques optiques du type d'objectif concerné, et **en ce que** le module de commande de laser (7) est conçu pour communiquer des instructions de commande en fonction des valeurs nominales physiques associées.

14. Dispositif (1) selon l'une des revendications 1 à 13, **caractérisé en ce que** l'identifiant de type d'objectif est réalisé sous la forme d'un parmi un identifiant mécanique, optique, électrique ou radioélectrique.

15. Dispositif (1) selon l'une des revendications 1 à 14, **caractérisé par** un système de mesure (9) pour déterminer la position par rapport au dispositif (1) de l'objectif de projection (3) raccordé au système de transmission optique (5).
